# EUROPEAN PATENT SPECIFICATION

(11) **EP 4 056 107 B1**
(45) Date of publication and mention of the grant of the patent: **26.03.2025**
(21) Application number: 20900178.3
(22) Date of filing: 09.12.2020
(51) Int. Cl.: A61B 5/022, A61B 5/107

(54) **WRIST-WORN ELECTRONIC DEVICE, METHOD FOR MEASURING WRIST SIZE, AND METHOD FOR MEASURING BLOOD PRESSURE**
AM HANDGELENK TRAGBARE ELEKTRONISCHE VORRICHTUNG, VERFAHREN ZUR MESSUNG DER HANDGELENKGRÖSSE UND VERFAHREN ZUR BLUTDRUCKMESSUNG
DISPOSITIF ÉLECTRONIQUE À PORTER AU POIGNET, PROCÉDÉ DE MESURE DE LA TAILLE D'UN POIGNET ET PROCÉDÉ DE MESURE DE LA PRESSION ARTÉRIELLE

(30) Priority: 13.12.2019 CN 201911284010
(43) Date of publication of application: 14.09.2022
(73) Proprietor: Huawei Technologies Co., Ltd., Shenzhen, Guangdong 518129 (CN)
(72) Inventor: WANG, Shaojian, Shenzhen, Guangdong 518129 (CN); HUANG, Zhenlong, Shenzhen, Guangdong 518129 (CN); LI, Jing, Shenzhen, Guangdong 518129 (CN); FU, Xiaoyu, Shenzhen, Guangdong 518129 (CN)
(74) Representative: MERH-IP Matias Erny Reichl Hoffmann Patentanwälte PartG mbB
(86) International application number: PCT/CN2020/134988
(87) International publication number: WO 2021/115342

(56) References cited:
- WO-A1-2019/160877
- CN-A- 1 500 440
- CN-A- 101 111 187
- CN-A- 108 403 102
- CN-A- 110 151 155
- CN-A- 110 151 185
- CN-A- 110 198 661
- CN-U- 205 285 120
- JP-A- H08 229 010
- US-A- 3 935 984
- US-A- 4 429 699
- US-A1- 2017 086 743
- BROCKWAY ROBERT ET AL: "Fully Implantable Arterial Blood Glucose Device for Metabolic Research Applications in Rats for Two Months", JOURNAL OF DIABETES SCIENCE AND TECHNOLOGY, vol. 9, no. 4, 27 May 2015 (2015-05-27), US, pages 771 - 781, XP093217368, ISSN: 1932-2968, Retrieved from the Internet <URL:https://journals.sagepub.com/doi/full-xml/10.1177/1932296815586424> DOI: 10.1177/1932296815586424

## Description

This application claims priority to Chinese Patent Application No. 201911284010.1, filed with the China National Intellectual Property Administration on December 13, 2019 and entitled "WRIST-WORN ELECTRONIC DEVICE, WRIST SIZE MEASUREMENT METHOD, AND BLOOD PRESSURE MEASUREMENT METHOD".

### TECHNICAL FIELD

This application relates to the field of wearable device technologies, and in particular, to a wrist-worn electronic device, a wrist size measurement method, and a blood pressure measurement method.

### BACKGROUND

With rapid development of electronic technologies and people's increasing attention to their own health status, wearable devices are increasingly widely used to assist in health assessment.

A blood pressure measurement apparatus is integrated into some wrist-worn electronic devices (for example, a watch or a band) in a conventional technology, and can measure blood pressure of a target user. For example, an airbag and a pressure sensor connected to the airbag are disposed on a watch. The airbag may compress a radial artery of a wrist of the target user in an inflation and deflation process. Therefore, the pressure sensor extracts a pulse wave signal of the target user, and can obtain blood pressure of the target user based on the pulse wave signal.

The wrist-worn electronic device attracts attention due to portability. However, in the conventional technology, a blood pressure measurement result of the wrist-worn electronic device is not accurate enough.
Document US 4 429 699 A generally discloses a blood pressure measuring equipment comprising a sleeve with a chamber inflatable by fluid and a measurement transducer for determining the circumference of an arm to which the sleeve is attached. A pressure sensor is provided to detect pressure in the chamber and is electrically connected through electronic components with two analog stores for storage of measured pressure values representing systolic and diastolic blood pressure. An indicating control device is connected between the stores and a digital display unit and comprises a pressure value correcting device by means of which the pressure values can be corrected, in dependence on the circumference of the arm as determined by the transducer, during readout of the stores.
Document US 2017/086743 A1 generally discloses a wearable electronic device that includes a body, a housing component, a band operable to attach the body to a body part of a user, and a force sensor coupled to the housing component. The force sensor is operable to produce a force signal based on a force exerted between the body part of the user and the housing component. A processing unit of the wearable electronic device receives the force signal from the force sensor and determines the force exerted on the housing component based thereon. The processing unit may use that force to determine a tightness of the band, determine health information for the user, adjust determined force exerted on a cover glass, and/or to perform various other actions.
Document WO 2019/1608777 A1 generally discloses a wearable rapid pulse confirmation ("RPC") device includes a Doppler array, a screen, and a loud speaker. The Doppler array detects a change in blood velocity in an artery through the Doppler array and provides feedback through the screen and the loudspeaker.

### SUMMARY

The following describes this application from a plurality of aspects. For the following implementations and beneficial effects of the plurality of aspects, refer to each other. In particular, embodiments of the present application are defined by the independent claims. Additional features of embodiments of the application are presented in the dependent claims. Embodiments of the present application (or the present disclosure) provide a wrist-worn electronic device that measure blood pressure, and a blood pressure measurement method.

According to a first aspect, an implementation of this application provides a wrist-worn electronic device that can measure blood pressure, including a main body and a wrist strap connected to the main body. The wrist strap is configured to wear the main body on a wrist of a target user. The wrist-worn electronic device further includes: a wrist size determining part, configured to measure, by using the wrist strap of the wrist-worn electronic device, a use circumference of the wrist-worn electronic device that matches a wrist size of the target user, and determine the wrist size of the target user based on the use circumference of the wrist-worn electronic device; and a blood pressure determining part, configured to detect a pulse wave signal of the target user, measure measured blood pressure of the target user based on the pulse wave signal, and correct the measured blood pressure of the target user based on the wrist size of the target user, to obtain first corrected blood pressure of the target user.

According to an implementation of this application, the wrist-worn electronic device can provide the target user with blood pressure corrected based on the wrist size of the target user. This can improve accuracy of blood pressure measurement.

In some implementations, when the wrist size of the target user is greater than a first threshold, the blood pressure determining part subtracts a first specified value from the measured blood pressure to obtain the first corrected blood pressure; and when the wrist size of the target user is less than a second threshold, the blood pressure determining part adds a second specified value to the measured blood pressure to obtain the first corrected blood pressure.

In some implementations, the wrist size of the target user is a wrist circumference of the target user, an optional range of the first threshold is 165 mm to 190 mm, and an optional range of the second threshold is 125 mm to 150 mm.

In some implementations, an optional range of the first specified value is 12 mmHg to 25 mmHg, and an optional range of the second specified value is 1 mmHg to 5 mmHg.

In some implementations, the wrist strap includes a first wrist strap and a second wrist strap that are connected to opposite ends of the main body of the wrist-worn electronic device, a first buckle part is disposed on the first wrist strap, a plurality of second buckle parts that can be fastened to the first buckle part are disposed on the second wrist strap, and the use circumference of the wrist-worn electronic device can be adjusted by fastening different second buckle parts in the plurality of second buckle parts to the first buckle part. The wrist size determining part can detect a second buckle part fastened to the first buckle part in the plurality of second buckle parts, to determine the use circumference of the wrist-worn electronic device.

According to an implementation of this application, measurement of the use circumference of the wrist-worn electronic device is converted into measurement of the second buckle part fastened to the first buckle part. This simplifies a process of measuring the use circumference of the wrist-worn electronic device.

In some implementations, the wrist-worn electronic device includes a power supply and a plurality of detection resistors disposed on the second wrist strap. At least one detection resistor is disposed between adjacent second buckle parts. When one of the plurality of second buckle parts is fastened to the first buckle part, a detection resistor between the second buckle part and the main body can form a closed loop with the power supply. The wrist size determining part can determine, based on a physical parameter corresponding to a resistance of the detection resistor in the closed loop, the second buckle part fastened to the first buckle part in the plurality of second buckle parts.

In some implementations, the wrist-worn electronic device further includes a pressure sensor disposed on an inner circumferential surface of the wrist-worn electronic device. The pressure sensor is configured to measure pressure between the wrist-worn electronic device and the wrist of the target user when the wrist-worn electronic device is worn on the wrist of the target user. The wrist size determining part determines the wrist size of the target user based on the use circumference of the wrist-worn electronic device and the pressure measured by the pressure sensor.

According to an implementation of this application, when determining the wrist size of the target user, the wrist size determining part compensates for a measurement deviation caused by wearing tightness. In this way, when wearing the wrist-worn electronic device, the user can adjust the use circumference of the wrist-worn electronic device based on a personal preference to comfortably wear the wrist-worn electronic device, and does not need to meet a specific wearing requirement. This improves user experience.

In some implementations, the wrist-worn electronic device further includes a pressure sensor disposed on an inner circumferential surface of the wrist-worn electronic device. The pressure sensor is configured to measure pressure between the wrist-worn electronic device and the wrist of the target user when the wrist-worn electronic device is worn on the wrist of the target user. The blood pressure determining part can correct the measured blood pressure of the target user based on the pressure measured by the pressure sensor and the wrist size of the target user, to obtain second corrected blood pressure of the target user.

According to an implementation of this application, in addition to correcting the measured blood pressure based on the wrist size of the target user, the blood pressure determining part further corrects the measured blood pressure based on the pressure measured by the pressure sensor. This further improves accuracy of blood pressure measurement.

According to a second aspect, an implementation of this application provides a wrist-worn electronic device, including a main body and a wrist strap connected to the main body. The wrist strap is configured to wear the main body on a wrist of a target user, and the wrist-worn electronic device further includes: a wrist size determining part, configured to measure, by using the wrist strap of the wrist-worn electronic device, a use circumference of the wrist-worn electronic device that matches a wrist size of the target user, and determine the wrist size of the target user based on the use circumference of the wrist-worn electronic device.

According to an implementation of this application, the wrist size determining part can measure the wrist size of the target user, so that the wrist-worn electronic device can correct measured blood pressure based on the wrist size of the user. This improves accuracy of blood pressure measurement.

In some implementations, the wrist strap includes a first wrist strap and a second wrist strap that are connected to opposite ends of the main body of the wrist-worn electronic device, a first buckle part is disposed on the first wrist strap, a plurality of second buckle parts that can be fastened to the first buckle part are disposed on the second wrist strap, and the use circumference of the wrist-worn electronic device can be adjusted by fastening different second buckle parts in the plurality of second buckle parts to the first buckle part. The wrist size determining part can detect a second buckle part fastened to the first buckle part in the plurality of second buckle parts, to determine the use circumference of the wrist-worn electronic device.

According to an implementation of this application, measurement of the use circumference of the wrist-worn electronic device is converted into measurement of the second buckle part fastened to the first buckle part. This simplifies a process of measuring the use circumference of the wrist-worn electronic device.

In some implementations, the wrist-worn electronic device includes a power supply and a plurality of detection resistors disposed on the second wrist strap. At least one detection resistor is disposed between adjacent second buckle parts. When one of the plurality of second buckle parts is fastened to the first buckle part, a detection resistor between the second buckle part and the main body can form a closed loop with the power supply. The wrist size determining part can determine, based on a physical parameter corresponding to a resistance of the detection resistor in the closed loop, the second buckle part fastened to the first buckle part in the plurality of second buckle parts.

In some implementations, the wrist size determining part further includes a pressure sensor disposed on an inner circumferential surface of the wrist-worn electronic device. The pressure sensor is configured to measure pressure between the wrist-worn electronic device and the wrist of the target user when the wrist-worn electronic device is worn on the wrist of the target user. The wrist size determining part determines the wrist size of the target user based on the use circumference of the wrist-worn electronic device and the pressure measured by the pressure sensor.

According to an implementation of this application, when determining the wrist size of the target user, the wrist size determining part compensates for a measurement deviation caused by wearing tightness. In this way, when wearing the wrist-worn electronic device, the user can adjust the use circumference of the wrist-worn electronic device based on a personal preference to comfortably wear the wrist-worn electronic device, and does not need to meet a specific wearing requirement. This improves user experience.

According to a third aspect, an implementation of this application provides a blood pressure measurement method, performed by a wrist-worn electronic device. The wrist-worn electronic device includes a main body and a wrist strap connected to the main body. The wrist strap is configured to wear the main body on a wrist of a target user. The method includes: measuring, by using the wrist strap of the wrist-worn electronic device, a use circumference of the wrist-worn electronic device that matches a wrist size of the target user, and determining the wrist size of the target user based on the use circumference of the wrist-worn electronic device; detecting a pulse wave signal of the target user, and determining measured blood pressure of the target user based on the pulse wave signal; and correcting the measured blood pressure of the target user based on the wrist size of the target user, to obtain first corrected blood pressure of the target user.

According to an implementation of this application, the wrist-worn electronic device can provide the target user with blood pressure corrected based on the wrist size of the target user. This can improve accuracy of blood pressure measurement.

In some implementations, the correcting the measured blood pressure of the target user based on the wrist size of the target user, to obtain first corrected blood pressure of the target user includes: when the wrist size of the target user is greater than a first threshold, subtracting the first specified value from the measured blood pressure to obtain the first corrected blood pressure; or when the wrist size of the target user is less than a second threshold, adding the second specified value to the measured blood pressure to obtain the first corrected blood pressure.

In some implementations, the wrist size of the target user is a wrist circumference of the target user, an optional range of the first threshold is 165 mm to 190 mm, and an optional range of the second threshold is 125 mm to 150 mm.

In some implementations, an optional range of the first specified value is 12 mmHg to 25 mmHg, and an optional range of the second specified value is 1 mmHg to 5 mmHg.

In some implementations, the wrist strap includes a first wrist strap and a second wrist strap that are connected to opposite ends of the main body of the wrist-worn electronic device, a first buckle part is disposed on the first wrist strap, a plurality of second buckle parts that can be fastened to the first buckle part are disposed on the second wrist strap, and the use circumference of the wrist-worn electronic device can be adjusted by fastening different second buckle parts in the plurality of second buckle parts to the first buckle part; and the measuring a use circumference of the wrist-worn electronic device that matches a wrist size of the target user, and determining the wrist size of the target user based on the use circumference of the wrist-worn electronic device includes: detecting a second buckle part fastened to the first buckle part in the plurality of second buckle parts, to determine the use circumference of the wrist-worn electronic device.

According to an implementation of this application, measurement of the use circumference of the wrist-worn electronic device is converted into measurement of the second buckle part fastened to the first buckle part. This simplifies a process of measuring the use circumference of the wrist-worn electronic device.

In some implementations, the wrist-worn electronic device includes a power supply and a plurality of detection resistors disposed on the second wrist strap. At least one detection resistor is disposed between adjacent second buckle parts. When one of the plurality of second buckle parts is fastened to the first buckle part, a detection resistor between the second buckle part and the main body can form a closed loop with the power supply; and the detecting a second buckle part fastened to the first buckle part in the plurality of second buckle parts, to determine the use circumference of the wrist-worn electronic device includes: determining, based on a physical parameter corresponding to a resistance of the detection resistor in the closed loop, the second buckle part fastened to the first buckle part in the plurality of second buckle parts.

In some implementations, the wrist-worn electronic device further includes a pressure sensor disposed on an inner circumferential surface of the wrist-worn electronic device. The pressure sensor is configured to measure pressure between the wrist-worn electronic device and the wrist of the target user when the wrist-worn electronic device is worn on the wrist of the target user; and the measuring a use circumference of the wrist-worn electronic device that matches a wrist size of the target user, and determining the wrist size of the target user based on the use circumference of the wrist-worn electronic device includes: determining the wrist size of the target user based on the use circumference of the wrist-worn electronic device and the pressure measured by the pressure sensor.

According to an implementation of this application, when determining the wrist size of the target user, a measurement deviation caused by wearing tightness is compensated. In this way, when wearing the wrist-worn electronic device, the user can adjust the use circumference of the wrist-worn electronic device based on a personal preference to comfortably wear the wrist-worn electronic device, and does not need to meet a specific wearing requirement. This improves user experience.

In some implementations, the wrist-worn electronic device further includes a pressure sensor disposed on an inner circumferential surface of the wrist-worn electronic device. The pressure sensor is configured to measure pressure between the wrist-worn electronic device and the wrist of the target user when the wrist-worn electronic device is worn on the wrist of the target user; and the correcting the measured blood pressure of the target user based on the wrist size of the target user, to obtain first corrected blood pressure of the target user includes: correcting the measured blood pressure of the target user based on the pressure measured by the pressure sensor and the wrist size of the target user, to obtain second corrected blood pressure of the target user.

According to an implementation of this application, the measured blood pressure is corrected based on the wrist size of the target user, and the measured blood pressure is further corrected based on the pressure measured by the pressure sensor. This further improves accuracy of blood pressure measurement.

According to a fourth aspect, an implementation of this application provides a wrist size measurement method, performed a wrist-worn electronic device. The wrist-worn electronic device includes a main body and a wrist strap connected to the main body. The wrist strap is configured to wear the main body on a wrist of a target user. The method further includes: measuring, by using the wrist strap of the wrist-worn electronic device, a use circumference of the wrist-worn electronic device that matches a wrist size of the target user, and determining the wrist size of the target user based on the use circumference of the wrist-worn electronic device.

According to an implementation of this application, the wrist size of the target user can be measured, so that the wrist-worn electronic device can correct measured blood pressure based on the wrist size of the user. This improves accuracy of blood pressure measurement.

In some implementations, the wrist strap includes a first wrist strap and a second wrist strap that are connected to opposite ends of the main body of the wrist-worn electronic device, a first buckle part is disposed on the first wrist strap, a plurality of second buckle parts that can be fastened to the first buckle part are disposed on the second wrist strap, and a use circumference of the wrist strap can be adjusted by fastening different second buckle parts in the plurality of second buckle parts to the first buckle part; and the measuring a use circumference of the wrist-worn electronic device that matches a wrist size of the target user, and determining the wrist size of the target user based on the use circumference of the wrist-worn electronic device includes: detecting a second buckle part fastened to the first buckle part in the plurality of second buckle parts, to determine the use circumference of the wrist-worn electronic device.

According to an implementation of this application, measurement of the use circumference of the wrist-worn electronic device is converted into measurement of the second buckle part fastened to the first buckle part. This simplifies a process of measuring the use circumference of the wrist-worn electronic device.

In some implementations, the wrist-worn electronic device includes a power supply and a plurality of detection resistors disposed on the second wrist strap. At least one detection resistor is disposed between adjacent second buckle parts. When one of the plurality of second buckle parts is fastened to the first buckle part, a detection resistor between the second buckle part and the main body can form a closed loop with the power supply; and the detecting a second buckle part fastened to the first buckle part in the plurality of second buckle parts, to determine the use circumference of the wrist-worn electronic device includes: determining, based on a physical parameter corresponding to a resistance of the detection resistor in the closed loop, the second buckle part fastened to the first buckle part in the plurality of second buckle parts.

In some implementations, the wrist-worn electronic device further includes a pressure sensor disposed on an inner circumferential surface of the wrist-worn electronic device. The pressure sensor is configured to measure pressure between the wrist-worn electronic device and the wrist of the target user when the wrist-worn electronic device is worn on the wrist of the target user; and the measuring a use circumference of the wrist-worn electronic device that matches a wrist size of the target user, and determining the wrist size of the target user based on the use circumference of the wrist-worn electronic device includes: determining the wrist size of the target user based on the use circumference of the wrist-worn electronic device and the pressure measured by the pressure sensor.

According to an implementation of this application, when determining the wrist size of the target user, a measurement deviation caused by wearing tightness is compensated. In this way, when wearing the wrist-worn electronic device, the user can adjust the use circumference of the wrist-worn electronic device based on a personal preference to comfortably wear the wrist-worn electronic device, and does not need to meet a specific wearing requirement. This improves user experience.

### BRIEF DESCRIPTION OF DRAWINGS

FIG. 1 is a schematic diagram of a structure of a watch according to an embodiment of this application;
FIG. 2 shows a front view of a watch according to an embodiment of this application;
FIG. 3 shows a side view of a watch according to an embodiment of this application;
FIG. 4 shows a rear view of a watch according to an embodiment of this application;
FIG. 5 is a diagram of a use status of a watch according to an embodiment of this application;
FIG. 6 shows an example of a relationship between a wrist size of a user and a deviation value of measured blood pressure;
FIG. 7 shows an example of a relationship between wearing tightness of a watch and a deviation value of measured blood pressure;
FIG. 8 shows a wrist size measurement method according to an embodiment of this application; and
FIG. 9 shows a blood pressure measurement method according to an embodiment of this application.

### DESCRIPTION OF EMBODIMENTS

The following describes implementations of this application by using specific embodiments. A person skilled in the art may easily learn of other advantages and effects of this application based on content disclosed in this specification. Although this application is described with reference to an example embodiment, it does not mean that a characteristic of this application is limited only to this implementation. On the contrary, a purpose of describing this application with reference to an implementation is to cover another option or modification that may be derived based on claims of this application. To provide an in-depth understanding of this application, the following descriptions include a plurality of specific details. This application may be alternatively implemented without using these details. In addition, to avoid confusion or blurring the focus of this application, some specific details will be omitted from the description. It should be noted that, when there is no conflict, embodiments in this application and the features in embodiments may be mutually combined.

It should be noted that, in this specification, reference numerals and letters in the following accompanying drawings represent similar items. Therefore, once an item is defined in an accompanying drawing, the item does not need to be further defined or interpreted in subsequent accompanying drawings.

In the description of the embodiments, it should be noted that, orientations or position relationships indicated by the terms "on", "in", and the like are orientations or position relationships shown based on the accompanying drawings, or orientations or position relationships that are commonly placed when the product in this application is used, and are merely intended to facilitate description of this application and simplify description, instead of indicating or implying that an indicated apparatus or element needs to have a specific orientation and be constructed and operated in a specific orientation, and therefore cannot be understood as limitations on this application.

The terms "first", "second", and the like are merely used for distinction and description, and shall not be understood as an indication or implication of relative importance.

In the descriptions of the embodiments, it should be further noted that, unless otherwise specified and limited, the terms "dispose" and "connect" should be understood in a broad sense. For example, a "connection" may be a fixed connection, a detachable connection, or an integrated connection; may be a mechanical connection or an electrical connection; may be a direct connection, or may be an indirect connection through an intermediate medium, or may be an internal connection between two components. The specific meanings about the foregoing terms in the embodiments may be understood by a person of ordinary skill in the art based on specific circumstances.

A wrist-worn electronic device that provides a blood pressure measurement function can detect a pulse wave signal at a wrist of a person, and detect blood pressure based on the pulse wave signal. Different wrist sizes have different impact on the blood pressure measured by the watch. To be specific, for a user with a large wrist size, measured blood pressure of the wrist-worn electronic device is greater than actual blood pressure of the user due to much fat on a wrist. For a user with a small wrist size, measured blood pressure of the wrist-worn electronic device is less than actual blood pressure of the user due to little fat on a wrist.

Based on the foregoing findings, an embodiment of this application provides a wrist-worn electronic device that can measure blood pressure, so that blood pressure measured based on a pulse wave can be corrected based on a wrist size of a target user. In this specification, the "target user" is a current user wearing the watch. The wrist-worn electronic device may be a watch, a band, or another electronic device that is worn on a wrist in a manner of wearing a watch. The following describes the technical solutions of this application by using a watch as an example.

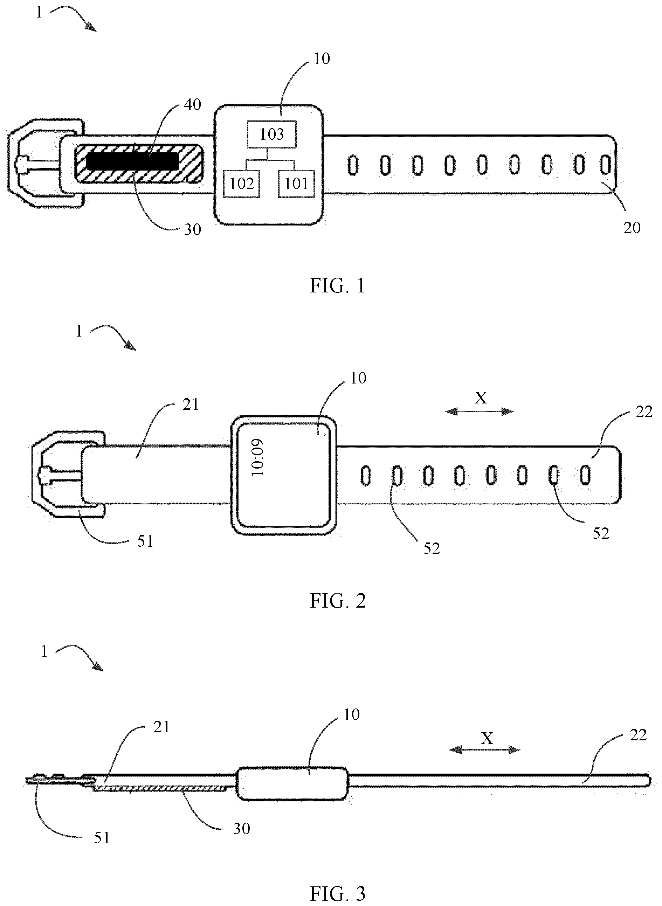

Specifically, as shown in FIG. 1, a watch 1 includes a watch body 10, a watch strap 20, and an airbag 30 and a pressure sensor 40 that are disposed on the watch strap 20. The pressure sensor 40 is configured to measure pressure between the watch 1 and a wrist of a target user. A wrist size determining part 101, a pressure measurement part 102, and a blood pressure determining part 103 are disposed inside the watch body 10. The wrist size determining part 101 can measure parameters from the watch strap 20 that are used to indicate a wrist size (for example, a wrist circumference, and a wrist diameter) of the target user, and determine the wrist size of the user based on the parameters. The blood pressure determining part 103 includes a pulse wave signal detection unit, determines blood pressure of the target user based on a detected pulse wave signal, and can correct measured blood pressure based on the wrist size of the target user to obtain corrected blood pressure. Optionally, the pressure measurement part 102 is configured to collect a pressure from the pressure sensor 40, and output the pressure to the blood pressure determining part 103, to further correct the blood pressure that is corrected based on the wrist size. The watch 1 in this embodiment can provide the target user with blood pressure corrected based on the wrist size of the target user and the pressure measured by the pressure sensor 40. This can improve accuracy of blood pressure measurement.

When the watch 1 is used, the target user may adjust, by adjusting an effective use length of the watch strap, a circumference of the watch 1 to match the wrist size of the target user, so that the watch 1 is worn comfortably. In this specification, the circumference of the watch 1 that matches the wrist size of the target user is referred to as "a use circumference of the watch". In this embodiment, the wrist size determining part 101 is configured to measure the use circumference of the watch by using the watch strap, and determine the wrist size of the target user based on the use circumference of the watch. For example, the wrist size determining part 101 may determine a wrist circumference of the target user as a value that is the same as the use circumference of the watch. In addition, the wrist size determining part 101 may be software, hardware, or a combination of software and hardware.

Specifically, refer to FIG. 1 to FIG. 5. The watch 1 according to implementations of this application includes the watch body 10 and the watch strap 20. The watch strap 20 includes a first watch strap 21 and a second watch strap 22 that are connected to two opposite ends of the watch body 10. A first buckle part 51 is disposed on the first watch strap 21, and a plurality of second buckle parts 52 that can be fastened to the first buckle part 51 are disposed on the second watch strap 22. Different second buckle parts 52 in the plurality of second buckle parts 52 are fastened to the first buckle part 51, to adjust an effective use length of the watch strap, and further adjust the use circumference of the watch.

Forms of the first buckle part 51 and the second buckle part 52 are not limited in this embodiment. For example, the first buckle part 51 and the second buckle part 52 may be buckle parts that can be fastened to each other in a folding buckle, a pin buckle, a press buckle, a butterfly buckle, a hook, or the like. Optionally, the plurality of second buckle parts 52 are distributed at equal intervals along a length direction (an X direction shown in FIG. 2 to FIG. 5) of the second watch strap 22.

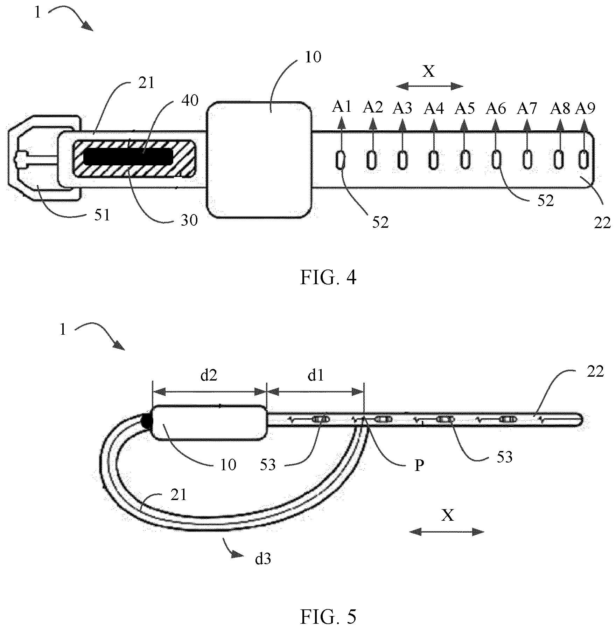

When the watch 1 is being worn, the wrist size determining part 101 can detect a second buckle part 52 fastened to the first buckle part 51 in the plurality of second buckle parts 52 (for ease of description, the second buckle part 52 fastened to the first buckle part 51 in the plurality of second buckle parts 52 is referred to as a "target second buckle part P" for short), to determine the use circumference of the watch. For example, refer to FIG. 5. A length d1 of an effective use section (a part that is of the second watch strap 22 and that is located between the watch body 10 and the target second buckle part P) of the second watch strap 22 is obtained by detecting a location of the target second buckle part P, and then the use circumference of the watch is obtained by accumulating the length d1 of the effective use section of the second watch strap 22, a length d2 of the watch body 10, a length d3 of the first watch strap 21, and the like. In this embodiment, measurement of the use circumference of the watch is converted into measurement of the target second buckle part P. This simplifies a measurement process of the use circumference of the watch 1.

Based on the technical solution provided in this embodiment, Table 1 shows an example of a correspondence between the location of the target second buckle part P and the use circumference of the watch. For locations represented by A1 to A9, refer to FIG. 4.

**Table 1**

| Location of the target second buckle part | Use circumference of the watch (mm) | Location of the target second buckle part | Use circumference of the watch (mm) |
|---|---|---|---|
| A1 | 137 | A6 | 180 |
| A2 | 144 | A7 | 190 |
| A3 | 152 | A8 | 200 |
| A4 | 161 | A9 | 211 |
| A5 | 170 | | |

Refer to FIG. 5. In this embodiment, the watch 1 includes a power supply (not shown) and a plurality of detection resistors 53 disposed on the second watch strap 22. When the first buckle part 51 is fastened to the second buckle part 52, a detection resistor 53 on the effective use section of the second watch strap 22 and the power supply may jointly form a closed length detection loop. To be specific, in this embodiment, the first buckle part 51 and the second buckle part 52 form a switch of the length detection loop. When the first buckle part 51 is fastened to the second buckle part 52, the length detection loop is closed. When the first buckle part 51 and the second buckle part 52 are detached, the length detection loop is open. Based on this, an implementation form of the switch is not limited in this application. For example, a material of the first buckle part 51 and a material of the second buckle part 52 are both conductive materials, and when the first buckle part 51 is fastened to the second buckle part 52, the length detection loop can be closed. Alternatively, a conductive sheet is disposed on each of the first buckle part 51 and the second buckle part 52, and when the first buckle part 51 is fastened to the second buckle part 52, the conductive sheet on the first buckle part 51 contacts the conductive sheet on the second buckle part 52, and therefore, the length detection loop is closed.

A form of the power supply is not limited in this embodiment. For example, the power supply may be a lithium battery, a power supply chip, or the like, provided that the power supply can supply power to the detection resistor 53. A location at which the power supply is disposed is not limited in this embodiment. The power supply may be disposed on the watch body 10, the first watch strap 21, or the second watch strap 22, provided that the power supply can form a closed loop with the detection resistor 53.

At least one detection resistor 53 is disposed between adjacent second buckle parts 52. Therefore, when different second buckle parts 52 are fastened to the first buckle part 51, a detection resistor 53 (briefly referred to as an "effective resistor") on the effective use section of the second watch strap 22 has different resistances (the resistance is a total resistance of the foregoing effective resistors, and is briefly referred to as an "effective resistance"). Optionally, the detection resistors 53 between the adjacent second buckle parts 52 have a same resistance. Optionally, the detection resistors 53 are connected in series with each other.

In this embodiment, the wrist size determining part 101 can determine a location of the target second buckle part P based on a physical parameter corresponding to a resistance of an effective resistor, to determine the use circumference of the watch 1. To be specific, in this embodiment, the physical parameter is used as a parameter indicating the use circumference of the watch, and the use circumference of the watch is obtained based on the physical parameter. A type of the physical parameter is not limited in this application. A person skilled in the art may determine a specific physical parameter based on a type of the length detection loop, for example, divided voltages at two ends of the effective resistor, a current passing through the effective resistor, and an effective resistance, provided that the physical parameter can correspond to the effective resistance.

The wrist size determining part 101 may further determine the location of the target second buckle part P in another manner. For example, in another embodiment, a pressure sensor is disposed on each second buckle part 52. It can be understood that, compared with the second buckle part 52 that is not fastened to the first buckle part 51, a pressure sensor on the target second buckle part P may detect larger pressure. Therefore, the wrist size determining part 101 can determine the location of the target second buckle part P based on pressure detected by the pressure sensor on each second buckle part 52.

In still another embodiment, a detection chip configured to detect the location of the target second buckle part P is disposed in the watch body 10. The detection chip may be a dedicated chip configured to detect the location of the target second buckle part P, or may be a part of the wrist size determining part 101 in the watch 1. The first buckle part 51 is connected to a power supply pin of the detection chip, and each second buckle part 52 is connected to a different detection pin of the detection chip. When the first buckle part 51 is fastened to the second buckle part 52, the first buckle part 51 is electrically connected to the second buckle part 52. It can be understood that, when the watch 1 is being worn, a detection pin connected to the target second buckle part P has a high level, and another detection pin has a low level. Therefore, the wrist size determining part 101 determines the location of the target second buckle part P by obtaining a level status of each detection pin.

When the target user wears the watch 1, the watch 1 is adjusted to different tightness based on different personal preferences. Specifically, if the target user prefers to adjust the watch 1 to a tight state, the actual wrist size of the target user may be greater than the wrist size determined based on the use circumference of the watch. If the target user prefers to adjust the watch 1 to a loose state, the actual wrist size of the target user may be less than the wrist size determined based on the use circumference of the watch.

To reduce impact of an improper wearing manner on a measurement result, the pressure sensor 40 is disposed on an inner circumferential surface of the watch 1 provided in this embodiment. The pressure sensor 40 is configured to measure pressure between the watch 1 and the wrist of the target user, to indicate wearing tightness of the watch 1. Herein, the inner circumferential surface of the watch 1 is a surface that faces the wrist of the target user when the watch 1 is being worn. In this embodiment, the wrist size determining part 101 can determine the wrist size of the target user based on the use circumference of the watch and the pressure measured by the pressure sensor 40. To be specific, in an embodiment of this application, when determining the wrist size of the target user, the wrist size determining part 101 compensates for a measurement deviation caused by wearing tightness. In this way, when wearing the watch 1, the user can adjust the use circumference of the watch based on a personal preference to comfortably wear the watch, and does not need to meet a specific wearing requirement. This improves user experience.

Specifically, when the pressure measured by the pressure sensor 40 is large, the wrist size of the target user that is determined based on the use circumference of the watch may be increased accordingly, to obtain a final wrist size of the target user. When the pressure measured by the pressure sensor 40 is small, the wrist size of the target user that is determined based on the use circumference of the watch may be decreased accordingly, to obtain a final wrist size of the target user.

According to the technical solution provided in this embodiment, Table 2 shows an example of a correspondence between pressure measured by the pressure sensor 40 and wearing tightness of the watch 1. Specific values of P1 to P4 may be measured through an experiment with reference to wearing experience of the watch 1. The wrist size determining part 101 may add a wrist circumference adjustment value listed in Table 2 on the wrist circumference of the target user that is obtained based on the use circumference of the watch, to obtain the final wrist circumference of the target user.

For example, based on the examples provided in Table 1 and Table 2, if a location of the target second buckle part P is A4, and the pressure measured by the pressure sensor 40 is from P3 to P4, the wrist circumference of the target user determined by the wrist size determining part 101 may be 161 mm + 2 mm, that is, 163 mm.

**Table 2**

| Pressure measured by the pressure sensor | < P1 | P1 to P2 | P2 to P3 | P3 to P4 | > P4 |
|---|---|---|---|---|---|
| Wearing tightness | Loose | Moderately loose | Moderate | Moderately tight | Tight |
| Wrist circumference adjustment value (mm) | -4 to -6 | -1 to -3 | 0 | 1 to 3 | 4 to 6 |

Vibration of blood pressure, a blood flow velocity, a blood flow, and transmission of the deformation and vibration of the vascular wall in the vascular system as intermittent contraction and relaxation of the heart are collectively referred to as pulse waves. As described above, the blood pressure determining part 103 in this embodiment can obtain measured blood pressure of the target user based on the pulse wave signal of the target user. Based on this, a specific blood pressure measurement method is not limited in this application. In this embodiment, the blood pressure measurement method is an oscillographic method. Refer to FIG. 3 and FIG. 4. The airbag 30 and a pressure measurement apparatus (not shown) connected to the airbag 30 are disposed on the watch 1. The airbag 30 compresses a radial artery of the wrist of the target user in an inflation and deflation process. Therefore, the pressure measurement apparatus obtains the pulse wave signal of the target user, and the blood pressure determining part 103 can determine the measured blood pressure of the target user based on the pulse wave signal. In other embodiments, the blood pressure measurement method may be a photoplethysmography method, a volume clamp method, or the like.

As described above, in this embodiment, the blood pressure determining part 103 can further correct the measured blood pressure of the target user based on the wrist size of the target user, to obtain a first corrected blood pressure of the target user. This improves blood pressure measurement precision.

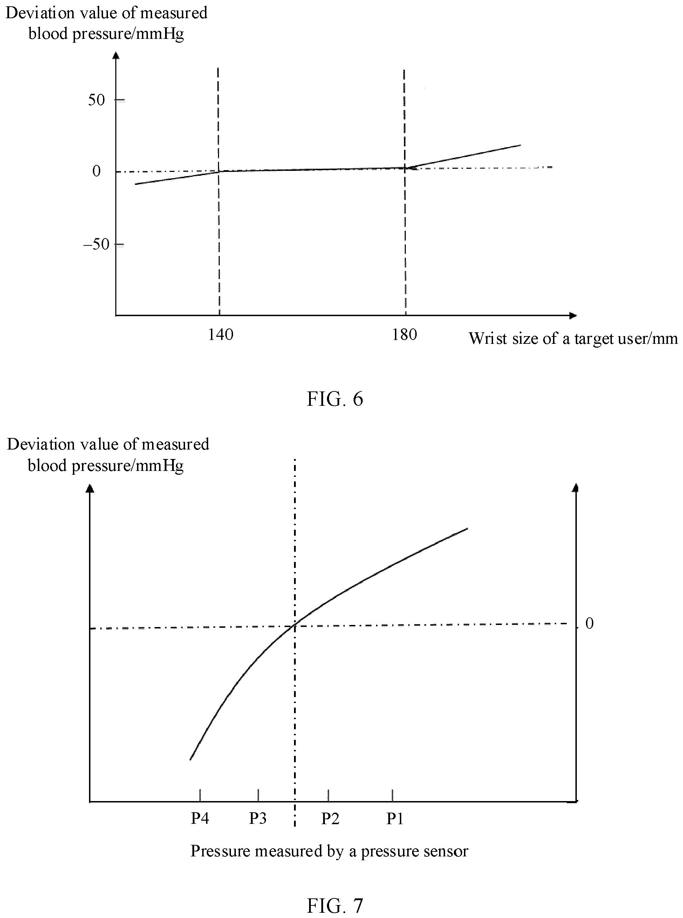

FIG. 6 shows an example of a relationship between the wrist size of the user and a deviation value (that is, a difference between the measured blood pressure of the target user and the actual blood pressure of the target user) of the measured blood pressure. For a target user with a large wrist size, measured blood pressure of the watch 1 is greater than an actual blood pressure of the user. Therefore, in this embodiment, when the wrist size of the target user is greater than a first threshold, the blood pressure determining part 103 subtracts a first specified value based on the measured blood pressure to obtain a first corrected blood pressure. For a target user with a small wrist size, measured blood pressure of the watch 1 is less than an actual blood pressure of the user. When the wrist size of the target user is less than a second threshold, the blood pressure determining part 103 adds a second specified value to the measured blood pressure to obtain a first corrected blood pressure.

In this embodiment, the wrist size of the target user is a wrist circumference of the target user. Correspondingly, based on an experiment result, an optional range of the first threshold is 165 mm to 190 mm, for example, 180 mm; and an optional range of the second threshold is 125 mm to 150 mm, for example, 140 mm.

Further, the first specified value and the second specified value may also be determined based on the experiment result. For example, an optional range of the first specified value is 12 mmHg to 25 mmHg, for example, 17 mmHg; and an optional range of the second specified value is 1 mmHg to 5 mmHg, for example, 3 mmHg.

In addition, different wearing tightness of the watch 1 also causes different deviations to the measured blood pressure of the watch 1. FIG. 7 shows an example of a relationship between wearing tightness of the watch 1 and a deviation value (that is, a difference between the measured blood pressure of the target user and the actual blood pressure of the target user) of the measured blood pressure. To be specific, if the watch 1 is worn extremely loosely, there is space between the airbag 30 and the skin, and atmospheric pressure in the airbag 30 is greater than pressure for compressing the skin. Consequently, a pulse wave signal moves towards high pressure, and the measured blood pressure is greater than the actual blood pressure of the user. If the airbag 30 is worn extremely tightly, atmospheric pressure in the airbag 30 is less than pressure for compressing the skin. Consequently, a pulse wave signal moves towards low pressure, and the measured blood pressure is less than the actual blood pressure of the user.

Therefore, in an embodiment of this application, in addition to correcting the measured blood pressure based on the wrist size of the target user, the blood pressure determining part 103 further corrects the measured blood pressure based on the pressure measured by the pressure sensor 40, to obtain second corrected blood pressure. This further improves accuracy of blood pressure measurement. For example, based on the pressure measured by the pressure sensor 40, a blood pressure adjustment value in Table 3 may be added to the first corrected blood pressure to obtain the second corrected blood pressure. A range of the blood pressure adjustment value may be determined based on the experiment. For example, based on the example provided in Table 3, when the pressure measured by the pressure sensor 40 is from P3 to P4, 10 mmHg may be added to the first corrected blood pressure to obtain the second corrected blood pressure.

**Table 3**

| Pressure measured by the pressure sensor | < P1 | P1 to P2 | P2 to P3 | P3 to P4 | > P4 |
|---|---|---|---|---|---|
| Wearing tightness | Loose | Moderately loose | Moderate | Moderately tight | Tight |
| Blood pressure adjustment value (mmHg) | -15 to -20 | -10 to -15 | 0 | 8 to 12 | 12 to 18 |

The following describes a wrist size measurement method and a blood pressure measurement method according to some embodiments. The methods can be implemented in the watch 1 described above in this specification. For content that is not described in the foregoing embodiments of watch 1, refer to the following method embodiments. Similarly, for content that is not described in the method embodiments, refer to the foregoing embodiments of watch 1.

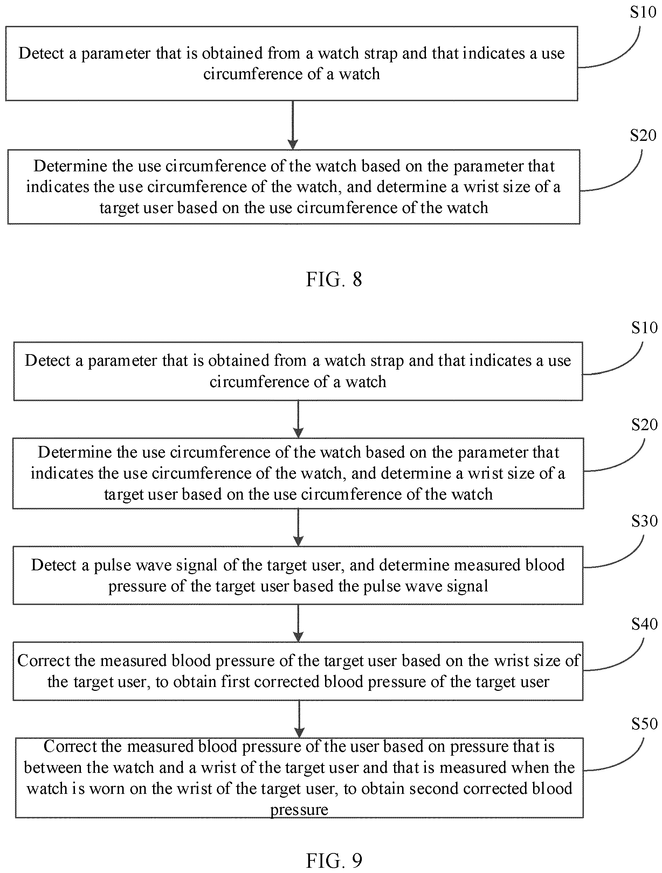

Refer to FIG. 8 and FIG. 1 to FIG. 5. An embodiment of this application provides a method for measuring a wrist size of a target user. The method is performed by the watch 1 provided in embodiments of this application, and specifically includes the following steps.

S10: Detect a parameter that indicates a use circumference of the watch and that is obtained from a watch strap, for example, divided voltages at two ends of an effective resistor (that is, a detection resistor 53 on an effective use section of a second watch strap 22), a current passing through the effective resistor, and an effective resistance.

S20: Determine the use circumference of the watch based on the parameter that indicates the use circumference of the watch, and determine the wrist size of the target user based on the use circumference of the watch.

In some possible implementations, the watch strap 20 includes a first watch strap 21 and the second watch strap 22 that are connected to two opposite ends of a watch body 10 of the watch 1. A first buckle part 51 is disposed on the first watch strap 21, and a plurality of second buckle parts 52 that can be fastened to the first buckle part 51 are disposed on the second watch strap 22. Different second buckle parts 52 in the plurality of second buckle parts 52 are fastened to the first buckle part 51, to adjust an effective use length of the watch strap 20. In step S10, the use circumference of the watch is determined by detecting a second buckle part 52 fastened to the first buckle part 51 in the plurality of second buckle parts 52. Forms of the first buckle part 51 and the second buckle part 52 are not limited in this embodiment. For example, the first buckle part 51 and the second buckle part 52 may be buckle parts that can be fastened to each other in a form of folding buckle, a pin buckle, a press buckle, a butterfly buckle, a hook, or the like.

In some optional implementations, the watch 1 includes a power supply and a plurality of detection resistors 53 disposed on the second watch strap 22. At least one detection resistor 53 is disposed between adjacent second buckle parts 52. When one of the plurality of second buckle parts 52 is fastened to the first buckle part 51, a detection resistor 53 between the second buckle part 52 and the watch body 10 can form a closed loop with the power supply. In step S10, the second buckle part 52 fastened to the first buckle part 51 in the plurality of second buckle parts 52 is determined based on a physical parameter corresponding to a resistance value of the detection resistor 53 in the closed loop.

In some optional implementations, the watch 1 further includes a pressure sensor 40 disposed on an inner circumferential surface of the watch 1. The pressure sensor 40 is configured to measure pressure between the watch 1 and a wrist of the target user when the watch 1 is worn on the wrist of the target user. In step S10, the wrist size of the target user is determined based on the use circumference of the watch and the pressure measured by the pressure sensor 40. When the pressure measured by the pressure sensor 40 is large, the wrist size of the target user that is determined based on the use circumference of the watch may be increased accordingly, to obtain a final wrist size of the target user. When the pressure measured by the pressure sensor 40 is small, the wrist size of the target user that is determined based on the use circumference of the watch may be decreased accordingly, to obtain a final wrist size of the target user.

Refer to FIG. 9 and FIG. 1 to FIG. 5. Another embodiment of this application provides a blood pressure measurement method. The method is performed by the watch 1 provided in embodiments of this application. The blood pressure measurement method provided in this embodiment is based on the method for measuring a wrist size of a target user in the previous embodiment. To be specific, the method in this embodiment includes step S10 and step S20 in the previous embodiment. Based on this, the method in this embodiment further includes the following steps.

S30: Detect a pulse wave signal of the target user, and determine measured blood pressure of the target user based on the pulse wave signal. In this embodiment, the blood pressure measurement method is an oscillographic method, an airbag 30 and a pressure measurement apparatus connected to the airbag 30 are disposed on the watch 1. The airbag 30 compresses a radial artery of a wrist of the target user in an inflation and deflation process. Therefore, the pressure measurement apparatus obtains the pulse wave signal of the target user, and a blood pressure determining part 103 can determine the measured blood pressure of the target user based on the pulse wave signal. In other embodiments, the blood pressure measurement method may be a photoplethysmography method, a volume clamp method, or the like.

S40: Correct the measured blood pressure of the target user based on the wrist size of the target user, to obtain a first corrected blood pressure of the target user.

In some implementations, in step S40, when the wrist size of the target user is greater than a first threshold, the blood pressure determining part 103 subtracts a first specified value from the measured blood pressure to obtain the first corrected blood pressure; and when the wrist size of the target user is less than a second threshold, the blood pressure determining part 103 adds a second specified value to the measured blood pressure to obtain the first corrected blood pressure.

In some optional implementations, in step S30, the wrist size of the target user is a wrist circumference of the target user. An optional range of the first threshold is 165 mm to 190 mm, for example, 180 mm; and an optional range of the second threshold is 125 mm to 150 mm, for example, 140 mm.

In some optional implementations, in step S30, an optional range of the first specified value is 12 to 25 mmHg, for example, 17 mmHg; and an optional range of the second specified value is 1 to 5 mmHg, for example, 3 mmHg.

S50: Correct the measured blood pressure of the user based on pressure that is between the watch and the wrist of the target user and that is measured when the watch is worn on the wrist of the target user, to obtain second corrected blood pressure.

The watch 1 further includes a pressure sensor 40 disposed on an inner circumferential surface of the watch 1. The pressure sensor 40 is configured to measure the pressure between the watch 1 and the wrist of the target user when the watch 1 is worn on the wrist of the target user. As further correction based on a result obtained in step S40, in step S50, the measured blood pressure of the target user is corrected based on the pressure measured by the pressure sensor 40 and the wrist size of the target user, to obtain the second corrected blood pressure of the target user.

The previous describes the technical solutions of this application by using a watch as an example. Based on the foregoing described specific implementations of this application, a person skilled in the art may understand that the structure and the method described in this application are also applicable to a band or another electronic device that is worn on a wrist in a manner of wearing a watch.

In conclusion, the foregoing embodiments in this application are merely illustrative of principles and effects of this application, but are not intended to limit this application.

## Claims

1. A wrist-worn electronic device (1) configured to measure blood pressure, comprising a main body (10) and a wrist strap (20) connected to the main body (10), wherein the wrist strap (20) is configured to wear the main body (10) on a wrist of a target user, and the wrist-worn electronic device (1) further comprises:
a wrist size determining module (101), configured to measure, by using the wrist strap (20) of the wrist-worn electronic device (1), a use circumference of the wrist-worn electronic device (1) that matches a wrist size of the target user, and determine the wrist size of the target user based on the use circumference of the wrist-worn electronic device (1); and
a blood pressure determining module (103), configured to detect a pulse wave signal of the target user, measure a blood pressure of the target user based on the pulse wave signal, and correct the blood pressure of the target user based on the wrist size of the target user, to obtain first corrected blood pressure of the target user,
**characterised in that**
the wrist-worn electronic device (1) further comprises a pressure sensor (40) disposed on an inner circumferential surface of the wrist-worn electronic device (1), and the pressure sensor (40) is configured to measure pressure between the wrist-worn electronic device (1) and the wrist of the target user when the wrist-worn electronic device (1) is worn on the wrist of the target user;
the wrist size determining module (101) is configured to determine said wrist size of the target user based on the use circumference of the wrist-worn electronic device (1) and the pressure measured by the pressure sensor (40); and
the blood pressure determining module (103) is configured to correct the measured blood pressure of the target user based on the pressure measured by the pressure sensor (40) and said wrist size of the target user, to obtain second corrected blood pressure of the target user.

2. The wrist-worn electronic device (1) according to claim 1, wherein
when the wrist size of the target user is greater than a first threshold, the blood pressure determining part subtracts a first specified value from the measured blood pressure to obtain the first corrected blood pressure; and
when the wrist size of the target user is less than a second threshold, the blood pressure determining part adds a second specified value to the measured blood pressure to obtain the first corrected blood pressure.

3. The wrist-worn electronic device (1) according to claim 1 or 2, wherein the wrist strap (20) comprises a first wrist strap (21) and a second wrist strap (22) that are connected to opposite ends of the main body (10) of the wrist-worn electronic device (1), a first buckle component (51) is disposed on the first wrist strap (21), a plurality of second buckle components (52) that can be fastened to the first buckle component (51) are disposed on the second wrist strap (22), and the use circumference of the wrist-worn electronic device (1) can be adjusted by fastening different second buckle components (52) in the plurality of second buckle components (52) to the first buckle component (51); and
the wrist size determining module (101) is configured to detect a second buckle component (52) fastened to the first buckle component (51) in the plurality of second buckle components (52), to determine the use circumference of the wrist-worn electronic device (1).

4. The wrist-worn electronic device (1) according to claim 3, wherein the wrist-worn electronic device (1) comprises a power supply and a plurality of detection resistors disposed on the second wrist strap (22), at least one detection resistor is disposed between adjacent second buckle components (52), and when one of the plurality of second buckle components (52) is fastened to the first buckle component (51), a detection resistor between the second buckle component (52) and the main body (10) is forming a closed loop with the power supply; and
the wrist size determining module (101) is configured to determine the wrist size based on a physical parameter corresponding to a resistance of the detection resistor in the closed loop.

5. The wrist-worn electronic device (1) according to claim 3 or 4, wherein a conductive sheet is disposed on each of the first buckle component (51) and the second buckle component (52).

6. The wrist-worn electronic device (1) according to claim 1, further comprises:
a detection chip disposed in the main body (10);
the first buckle component (51) is connected to a power supply pin of the detection chip;
each second buckle component (52) is connected to a different detection pin of the detection chip, wherein when the first buckle component (51) is connected to a target second buckle component (P), a level status of the corresponding detection pin that connects to the target second buckle component (P) has a high level and a level status of a detection pin that does not connect to the target second buckle component (P) has a low level.

7. A blood pressure measurement method, performed by a wrist-worn electronic device, wherein the wrist-worn electronic device comprises a main body and a wrist strap connected to the main body, the wrist strap is configured to wear the main body on a wrist of a target user, and the method comprises:
measuring (S20), by using the wrist strap of the wrist-worn electronic device, a use circumference of the wrist-worn electronic device that matches a wrist size of the target user, and determining the wrist size of the target user based on the use circumference of the wrist-worn electronic device;
detecting (S30) a pulse wave signal of the target user, and determining measured blood pressure of the target user based on the pulse wave signal; and
correcting (S40) the measured blood pressure of the target user based on the wrist size of the target user, to obtain first corrected blood pressure of the target user,
wherein the wrist-worn electronic device further comprises a pressure sensor disposed on an inner circumferential surface of the wrist-worn electronic device, and the pressure sensor is configured to measure pressure between the wrist-worn electronic device and the wrist of the target user when the wrist-worn electronic device is worn on the wrist of the target user;
the measuring a use circumference of the wrist-worn electronic device that matches a wrist size of the target user, and determining the wrist size of the target user based on the use circumference of the wrist-worn electronic device comprises:
determining the wrist size of the target user based on the use circumference of the wrist-worn electronic device and the pressure measured by the pressure sensor; and
the correcting the measured blood pressure of the target user based on the wrist size of the target user, to obtain first corrected blood pressure of the target user comprises:
correcting (S50) the measured blood pressure of the target user based on the pressure measured by the pressure sensor and the wrist size of the target user, to obtain second corrected blood pressure of the target user.

8. The method according to claim 7, wherein the correcting (S40) the measured blood pressure of the target user based on the wrist size of the target user, to obtain first corrected blood pressure of the target user comprises:
when the wrist size of the target user is greater than a first threshold, subtracting a first specified value from the measured blood pressure to obtain the first corrected blood pressure; and
when the wrist size of the target user is less than a second threshold, adding a second specified value to the measured blood pressure to obtain the first corrected blood pressure.

9. The method according to claim 7, wherein the wrist strap comprises a first wrist strap and a second wrist strap that are connected to opposite ends of the main body, a first buckle part is disposed on the first wrist strap, a plurality of second buckle parts that can be fastened to the first buckle part are disposed on the second wrist strap, and the use circumference of the wrist-worn electronic device can be adjusted by fastening different second buckle parts in the plurality of second buckle parts to the first buckle part; and
the measuring (S20) a use circumference of the wrist-worn electronic device that matches a wrist size of the target user, and determining the wrist size of the target user based on the use circumference of the wrist-worn electronic device comprises:
detecting a second buckle part fastened to the first buckle part in the plurality of second buckle parts, to determine the use circumference of the wrist-worn electronic device.

10. The method according to claim 9, wherein the wrist-worn electronic device comprises a power supply and a plurality of detection resistors disposed on the second wrist strap, at least one detection resistor is disposed between adjacent second buckle parts, and when one of the plurality of second buckle parts is fastened to the first buckle part, a detection resistor between the second buckle part and the main body is forming a closed loop with the power supply; and
the detecting a second buckle part fastened to the first buckle part in the plurality of second buckle parts, to determine the use circumference of the wrist-worn electronic device comprises:
determining, based on a physical parameter corresponding to a resistance of the detection resistor in the closed loop, the second buckle part fastened to the first buckle part in the plurality of second buckle parts.

11. The method according to claim 7, wherein the electronic device comprises a detection chip disposed in the main body;
the first buckle component is connected to a power supply pin of the detection chip;
each second buckle component is connected to a different detection pin of the detection chip;
the method further comprises:
when the first buckle component is connected to a target second buckle component, determine the level status of each detection pin of the detection chip, wherein a level status of the corresponding detection pin that connects to the target second buckle component has a high level and a level status of a detection pin that does not connect to the target second buckle component has a low level.

## Patentansprüche

1. Am Handgelenk getragene elektronische Vorrichtung (1), die konfiguriert ist, um einen Blutdruck zu messen, die einen Hauptkörper (10) und ein Handgelenkband (20), das mit dem Hauptkörper (10) verbunden ist, umfasst, wobei das Handgelenkband (20) konfiguriert ist, um den Hauptkörper (10) an einem Handgelenk eines Zielbenutzers zu tragen, und die am Handgelenk getragene elektronische Vorrichtung (1) ferner umfasst:
ein Handgelenkgrößenbestimmungsmodul (101), das konfiguriert ist, um, durch Verwenden des Handgelenkbands (20) der am Handgelenk getragenen elektronischen Vorrichtung (1), einen Verwendungsumfang der am Handgelenk getragenen elektronischen Vorrichtung (1) zu messen, der mit einer Handgelenkgröße des Zielbenutzers übereinstimmt, und die Handgelenkgröße des Zielbenutzers basierend auf dem Verwendungsumfang der am Handgelenk getragenen elektronischen Vorrichtung (1) zu bestimmen; und
ein Blutdruckbestimmungsmodul (103), das konfiguriert ist, um ein Pulswellensignal des Zielbenutzers zu erfassen, einen Blutdruck des Zielbenutzers basierend auf dem Pulswellensignal zu messen und den Blutdruck des Zielbenutzers basierend auf der Handgelenkgröße des Zielbenutzers zu korrigieren, um einen ersten korrigierten Blutdruck des Zielbenutzers zu erhalten,
**dadurch gekennzeichnet, dass** die am Handgelenk getragene elektronische Vorrichtung (1) ferner einen Drucksensor (40), der an einer inneren Umfangsoberfläche der am Handgelenk getragenen elektronischen Vorrichtung (1) angeordnet ist, umfasst und der Drucksensor (40) konfiguriert ist, um einen Druck zwischen der am Handgelenk getragenen elektronischen Vorrichtung (1) und dem Handgelenk des Zielbenutzers zu messen, wenn die am Handgelenk getragene elektronische Vorrichtung (1) an dem Handgelenk des Zielbenutzers getragen wird;
das Handgelenkgrößenbestimmungsmodul (101) konfiguriert ist, um die Handgelenkgröße des Zielbenutzers basierend auf dem Verwendungsumfang der am Handgelenk getragenen elektronischen Vorrichtung (1) und dem Druck, der durch den Drucksensor (40) gemessen wird, zu bestimmen; und
das Blutdruckbestimmungsmodul (103) konfiguriert ist, um den gemessenen Blutdruck des Zielbenutzers basierend auf dem Druck, der durch den Drucksensor (40) gemessen wird, und der Handgelenkgröße des Zielbenutzers zu korrigieren, um einen zweiten korrigierten Blutdruck des Zielbenutzers zu erhalten.

2. Am Handgelenk getragene elektronische Vorrichtung (1) nach Anspruch 1, wobei, wenn die Handgelenkgröße des Zielbenutzers größer als eine erste Schwelle ist, das Blutdruckbestimmungsteil einen ersten angegebenen Wert von dem gemessenen Blutdruck subtrahiert, um den ersten korrigierten Blutdruck zu erhalten; und
wenn die Handgelenkgröße des Zielbenutzers kleiner als eine zweite Schwelle ist, das Blutdruckbestimmungsteil einen zweiten angegebenen Wert zu dem gemessenen Blutdruck addiert, um den ersten korrigierten Blutdruck zu erhalten.

3. Am Handgelenk getragene elektronische Vorrichtung (1) nach Anspruch 1 oder 2, wobei das Handgelenkband (20) ein erstes Handgelenkband (21) und ein zweites Handgelenkband (22) umfasst, die mit gegenüberliegenden Enden des Hauptkörpers (10) der am Handgelenk getragenen elektronischen Vorrichtung (1) verbunden sind, eine erste Schnallenkomponente (51) an dem ersten Handgelenkband (21) angeordnet ist, eine Vielzahl von zweiten Schnallenkomponenten (52), die an der ersten Schnallenkomponente (51) befestigt werden können, an dem zweiten Handgelenkband (22) angeordnet sind und der Verwendungsumfang der am Handgelenk getragenen elektronischen Vorrichtung (1) durch Befestigen unterschiedlicher zweiter Schnallenkomponenten (52) aus der Vielzahl von zweiten Schnallenkomponenten (52) an der ersten Schnallenkomponente (51) eingestellt werden kann; und
das Handgelenkgrößenbestimmungsmodul (101) konfiguriert ist, um eine zweite Schnallenkomponente (52), die an der ersten Schnallenkomponente (51) befestigt ist, aus der Vielzahl von zweiten Schnallenkomponenten (52) zu erfassen, um den Verwendungsumfang der am Handgelenk getragenen elektronischen Vorrichtung (1) zu bestimmen.

4. Am Handgelenk getragene elektronische Vorrichtung (1) nach Anspruch 3, wobei die am Handgelenk getragene elektronische Vorrichtung (1) eine Stromversorgung und eine Vielzahl von Erfassungswiderstandsbauelementen, die an dem zweiten Handgelenkband (22) angeordnet sind, umfasst, mindestens ein Erfassungswiderstandsbauelement zwischen angrenzenden zweiten Schnallenkomponenten (52) angeordnet ist und, wenn eine der Vielzahl von zweiten Schnallenkomponenten (52) an der ersten Schnallenkomponente (51) befestigt ist, ein Erfassungswiderstandsbauelement zwischen der zweiten Schnallenkomponente (52) und dem Hauptkörper (10) einen geschlossenen Kreislauf mit der Stromversorgung ausbildet; und
das Handgelenkgrößenbestimmungsmodul (101) konfiguriert ist, um die Handgelenkgröße basierend auf einem physikalischen Parameter, der einem Widerstand des Erfassungswiderstandsbauelements in dem geschlossenen Kreislauf entspricht, zu bestimmen.

5. Am Handgelenk getragene elektronische Vorrichtung (1) nach Anspruch 3 oder 4, wobei eine leitfähige Folie an jeder der ersten Schnallenkomponente (51) und der zweiten Schnallenkomponente (52) angeordnet ist.

6. Am Handgelenk getragene elektronische Vorrichtung (1) nach Anspruch 1, die ferner umfasst:
einen Erfassungschip, der in dem Hauptkörper (10) angeordnet ist;
wobei die erste Schnallenkomponente (51) mit einem Stromversorgungsstift des Erfassungschips verbunden ist;
wobei jede zweite Schnallenkomponente (52) mit einem unterschiedlichen Erfassungsstift des Erfassungschips verbunden ist, wobei, wenn die erste Schnallenkomponente (51) mit einer zweiten Zielschnallenkomponente (P) verbunden ist, ein Pegelstatus des entsprechenden Erfassungsstifts, der sich mit der zweiten Zielschnallenkomponente (P) verbindet, einen hohen Pegel aufweist und ein Pegelstatus eines Erfassungsstifts, der sich nicht mit der zweiten Zielschnallenkomponente (P) verbindet, einen niedrigen Pegel aufweist.

7. Blutdruckmessverfahren, das durch eine am Handgelenk getragene elektronische Vorrichtung durchgeführt wird, wobei die am Handgelenk getragene elektronische Vorrichtung einen Hauptkörper und ein Handgelenkband, das mit dem Hauptkörper verbunden ist, umfasst, das Handgelenkband konfiguriert ist, um den Hauptkörper an einem Handgelenk eines Zielbenutzers zu tragen, und das Verfahren umfasst:
Messen (S20), durch Verwenden des Handgelenkbands der am Handgelenk getragenen elektronischen Vorrichtung, eines Verwendungsumfangs der am Handgelenk getragenen elektronischen Vorrichtung, der mit einer Handgelenkgröße des Zielbenutzers übereinstimmt, und Bestimmen der Handgelenkgröße des Zielbenutzers basierend auf dem Verwendungsumfang der am Handgelenk getragenen elektronischen Vorrichtung;
Erfassen (S30) eines Pulswellensignals des Zielbenutzers und Bestimmen des gemessenen Blutdrucks des Zielbenutzers basierend auf dem Pulswellensignal; und
Korrigieren (S40) des gemessenen Blutdrucks des Zielbenutzers basierend auf der Handgelenkgröße des Zielbenutzers, um den ersten korrigierten Blutdruck des Zielbenutzers zu erhalten,
wobei die am Handgelenk getragene elektronische Vorrichtung ferner einen Drucksensor, der an einer inneren Umfangsoberfläche der am Handgelenk getragenen elektronischen Vorrichtung angeordnet ist, umfasst und der Drucksensor konfiguriert ist, um den Druck zwischen der am Handgelenk getragenen elektronischen Vorrichtung und dem Handgelenk des Zielbenutzers zu messen, wenn die am Handgelenk getragene elektronische Vorrichtung an dem Handgelenk des Zielbenutzers getragen wird;
das Messen eines Verwendungsumfangs der am Handgelenk getragenen elektronischen Vorrichtung, der mit einer Handgelenkgröße des Zielbenutzers übereinstimmt, und das Bestimmen der Handgelenkgröße des Zielbenutzers basierend auf dem Verwendungsumfang der am Handgelenk getragenen elektronischen Vorrichtung umfasst:
Bestimmen der Handgelenkgröße des Zielbenutzers basierend auf dem Verwendungsumfang der am Handgelenk getragenen elektronischen Vorrichtung und dem Druck, der durch den Drucksensor gemessen wird; und
das Korrigieren des gemessenen Blutdrucks des Zielbenutzers basierend auf der Handgelenkgröße des Zielbenutzers, um den ersten korrigierten Blutdruck des Zielbenutzers zu erhalten, umfasst:
Korrigieren (S50) des gemessenen Blutdrucks des Zielbenutzers basierend auf dem Druck, der durch den Drucksensor gemessen wird, und der Handgelenkgröße des Zielbenutzers, um den zweiten korrigierten Blutdruck des Zielbenutzers zu erhalten.

8. Verfahren nach Anspruch 7, wobei das Korrigieren (S40) des gemessenen Blutdrucks des Zielbenutzers basierend auf der Handgelenkgröße des Zielbenutzers, um den ersten korrigierten Blutdruck des Zielbenutzers zu erhalten, umfasst:
wenn die Handgelenkgröße des Zielbenutzers größer als eine erste Schwelle ist, Subtrahieren eines ersten angegebenen Werts von dem gemessenen Blutdruck, um den ersten korrigierten Blutdruck zu erhalten; und
wenn die Handgelenkgröße des Zielbenutzers kleiner als eine zweite Schwelle ist, Addieren eines zweiten angegebenen Werts zu dem gemessenen Blutdruck, um den ersten korrigierten Blutdruck zu erhalten.

9. Verfahren nach Anspruch 7, wobei das Handgelenkband ein erstes Handgelenkband und ein zweites Handgelenkband umfasst, die mit gegenüberliegenden Enden des Hauptkörpers verbunden sind, ein erstes Schnallenteil an dem ersten Handgelenkband angeordnet ist, eine Vielzahl von zweiten Schnallenteilen, die an dem ersten Schnallenteil befestigt werden können, an dem zweiten Handgelenkband angeordnet sind und der Verwendungsumfang der am Handgelenk getragenen elektronischen Vorrichtung durch Befestigen unterschiedlicher zweiter Schnallenteile aus der Vielzahl von zweiten Schnallenteilen an dem ersten Schnallenteil eingestellt werden kann; und das Messen (S20) eines Verwendungsumfangs der am Handgelenk getragenen elektronischen Vorrichtung, der mit einer Handgelenkgröße des Zielbenutzers übereinstimmt, und das Bestimmen der Handgelenkgröße des Zielbenutzers basierend auf dem Verwendungsumfang der am Handgelenk getragenen elektronischen Vorrichtung umfasst:
Erfassen eines zweiten Schnallenteils, das an dem ersten Schnallenteil befestigt ist, aus der Vielzahl von zweiten Schnallenteilen, um den Verwendungsumfang der am Handgelenk getragenen elektronischen Vorrichtung zu bestimmen.

10. Verfahren nach Anspruch 9, wobei die am Handgelenk getragene elektronische Vorrichtung eine Stromversorgung und eine Vielzahl von Erfassungswiderstandsbauelementen, die an dem zweiten Handgelenkband angeordnet sind, umfasst, mindestens ein Erfassungswiderstandsbauelement zwischen angrenzenden zweiten Schnallenteilen angeordnet ist, und, wenn eines der Vielzahl von zweiten Schnallenteilen an dem ersten Schnallenteil befestigt ist, ein Erfassungswiderstandsbauelement zwischen dem zweiten Schnallenteil und dem Hauptkörper einen geschlossenen Kreislauf mit der Stromversorgung ausbildet; und das Erfassen eines zweiten Schnallenteils, das an dem ersten Schnallenteil befestigt ist, aus der Vielzahl von zweiten Schnallenteilen, um den Verwendungsumfang der am Handgelenk getragenen elektronischen Vorrichtung zu bestimmen, umfasst:
Bestimmen, basierend auf einem physikalischen Parameter, der einem Widerstand des Erfassungswiderstandsbauelements in dem geschlossenen Kreislauf entspricht, des zweiten Schnallenteils, das an dem ersten Schnallenteil befestigt ist, aus der Vielzahl von zweiten Schnallenteilen.

11. Verfahren nach Anspruch 7, wobei die elektronische Vorrichtung einen Erfassungschip, der in dem Hauptkörper angeordnet ist, umfasst;
wobei die erste Schnallenkomponente mit einem Stromversorgungsstift des Erfassungschips verbunden ist;
wobei jede zweite Schnallenkomponente mit einem unterschiedlichen Erfassungsstift des Erfassungschips verbunden ist;
wobei das Verfahren ferner umfasst:
wenn die erste Schnallenkomponente mit einer zweiten Zielschnallenkomponente verbunden ist, Bestimmen des Pegelstatus jedes Erfassungsstifts des Erfassungschips, wobei ein Pegelstatus des entsprechenden Erfassungsstifts, der sich mit der zweiten Zielschnallenkomponente verbindet, einen hohen Pegel aufweist und ein Pegelstatus eines Erfassungsstifts, der sich nicht mit der zweiten Zielschnallenkomponente verbindet, einen niedrigen Pegel aufweist.

## Revendications

1. Dispositif électronique porté au poignet (1) configuré pour mesurer la pression artérielle, comprenant un corps principal (10) et un bracelet (20) relié au corps principal (10), dans lequel le bracelet (20) est conçu pour porter le corps principal (10) sur un poignet d'un utilisateur cible, et le dispositif électronique porté au poignet (1) comprend en outre :
un module de détermination de taille de poignet (101), configuré pour mesurer, en utilisant le bracelet (20) du dispositif électronique porté au poignet (1), une circonférence d'utilisation du dispositif électronique porté au poignet (1) qui s'adapte à une taille de poignet de l'utilisateur cible, et déterminer la taille de poignet de l'utilisateur cible sur la base de la circonférence d'utilisation du dispositif électronique porté au poignet (1) ; et
un module de détermination de pression artérielle (103), configuré pour détecter un signal d'onde pulsée de l'utilisateur cible, mesurer une pression artérielle de l'utilisateur cible sur la base du signal d'onde pulsée, et corriger la pression artérielle de l'utilisateur cible sur la base de la taille de poignet de l'utilisateur cible, afin d'obtenir une première pression artérielle corrigée de l'utilisateur cible,
**caractérisé en ce que** le dispositif électronique porté au poignet (1) comprend en outre un capteur de pression (40) disposé sur une surface circonférentielle interne du dispositif électronique porté au poignet (1), et le capteur de pression (40) est configuré pour mesurer une pression entre le dispositif électronique porté au poignet (1) et le poignet de l'utilisateur cible lorsque le dispositif électronique porté au poignet (1) est porté sur le poignet de l'utilisateur cible ;
le module de détermination de taille de poignet (101) est configuré pour déterminer ladite taille de poignet de l'utilisateur cible sur la base de la circonférence d'utilisation du dispositif électronique porté au poignet (1) et de la pression mesurée par le capteur de pression (40) ; et
le module de détermination de pression artérielle (103) est configuré pour corriger la pression artérielle mesurée de l'utilisateur cible sur la base de la pression mesurée par le capteur de pression (40) et de ladite taille de poignet de l'utilisateur cible, afin d'obtenir une seconde pression artérielle corrigée de l'utilisateur cible.

2. Dispositif électronique porté au poignet (1) selon la revendication 1, dans lequel lorsque la taille de poignet de l'utilisateur cible est supérieure à un premier seuil, la partie de détermination de pression artérielle soustrait une première valeur spécifiée de la pression artérielle mesurée pour obtenir la première pression artérielle corrigée ; et
lorsque la taille de poignet de l'utilisateur cible est inférieure à un second seuil, la partie de détermination de pression artérielle ajoute une seconde valeur spécifiée à la pression artérielle mesurée pour obtenir la première pression artérielle corrigée.

3. Dispositif électronique porté au poignet (1) selon la revendication 1 ou 2, dans lequel le bracelet (20) comprend un premier bracelet (21) et un second bracelet (22) qui sont reliés à des extrémités opposées du corps principal (10) du dispositif électronique porté au poignet (1), un premier composant de boucle (51) est disposé sur le premier bracelet (21), une pluralité de seconds composants de boucle (52) qui peuvent être fixés au premier composant de boucle (51) sont disposés sur le second bracelet (22), et la circonférence d'utilisation du dispositif électronique porté au poignet (1) peut être ajustée en fixant différents seconds composants de boucle (52) dans la pluralité de seconds composants de boucle (52) au premier composant de boucle (51) ; et
le module de détermination de taille de poignet (101) est configuré pour détecter un second composant de boucle (52) fixé au premier composant de boucle (51) dans la pluralité de seconds composants de boucle (52), afin de déterminer la circonférence d'utilisation du dispositif électronique porté au poignet (1).

4. Dispositif électronique porté au poignet (1) selon la revendication 3, dans lequel le dispositif électronique porté au poignet (1) comprend une alimentation électrique et une pluralité de résistances de détection disposées sur le second bracelet (22), au moins une résistance de détection est disposée entre des seconds composants de boucle (52) adjacents, et lorsque l'un parmi la pluralité de seconds composants de boucle (52) est fixé au premier composant de boucle (51), une résistance de détection entre le second composant de boucle (52) et le corps principal (10) forme une boucle fermée avec l'alimentation électrique ; et
le module de détermination de taille de poignet (101) est configuré pour déterminer la taille de poignet sur la base d'un paramètre physique correspondant à une résistance de la résistance de détection dans la boucle fermée.

5. Dispositif électronique porté au poignet (1) selon la revendication 3 ou 4, dans lequel une feuille conductrice est disposée sur chacun parmi le premier composant de boucle (51) et le second composant de boucle (52).

6. Dispositif électronique porté au poignet (1) selon la revendication 1, comprenant en outre :
une puce de détection disposée dans le corps principal (10) ;
le premier composant de boucle (51) est relié à une broche d'alimentation électrique de la puce de détection ;
chaque second composant de boucle (52) est relié à une broche de détection différente de la puce de détection, dans lequel, lorsque le premier composant de boucle (51) est relié à un second composant de boucle cible (P), un état de niveau de la broche de détection correspondante qui est reliée au second composant de boucle cible (P) présente un niveau élevé et un état de niveau d'une broche de détection qui n'est pas reliée au second composant de boucle cible (P) présente un niveau bas.

7. Procédé de mesure de pression artérielle, réalisé par un dispositif électronique porté au poignet, dans lequel le dispositif électronique porté au poignet comprend un corps principal et un bracelet relié au corps principal, le bracelet est conçu pour porter le corps principal sur un poignet d'un utilisateur cible, et le procédé comprend :
la mesure (S20), en utilisant le bracelet du dispositif électronique porté au poignet, d'une circonférence d'utilisation du dispositif électronique porté au poignet qui s'adapte à une taille de poignet de l'utilisateur cible, et la détermination de la taille de poignet de l'utilisateur cible sur la base de la circonférence d'utilisation du dispositif électronique porté au poignet ;
la détection (S30) d'un signal d'onde pulsée de l'utilisateur cible, et la détermination de la pression artérielle mesurée de l'utilisateur cible sur la base du signal d'onde pulsée ; et
la correction (S40) de la pression artérielle mesurée de l'utilisateur cible sur la base de la taille de poignet de l'utilisateur cible, afin d'obtenir une première pression artérielle corrigée de l'utilisateur cible,
dans lequel le dispositif électronique porté au poignet comprend en outre un capteur de pression disposé sur une surface circonférentielle interne du dispositif électronique porté au poignet, et le capteur de pression est configuré pour mesurer une pression entre le dispositif électronique porté au poignet et le poignet de l'utilisateur cible lorsque le dispositif électronique porté au poignet est porté sur le poignet de l'utilisateur cible ;
la mesure d'une circonférence d'utilisation du dispositif électronique porté au poignet qui s'adapte à une taille de poignet de l'utilisateur cible, et la détermination de la taille de poignet de l'utilisateur cible sur la base de la circonférence d'utilisation du dispositif électronique porté au poignet comprend :
la détermination de la taille de poignet de l'utilisateur cible sur la base de la circonférence d'utilisation du dispositif électronique porté au poignet et de la pression mesurée par le capteur de pression ; et
la correction de la pression artérielle mesurée de l'utilisateur cible sur la base de la taille de poignet de l'utilisateur cible, afin d'obtenir une première pression artérielle corrigée de l'utilisateur cible comprend :
la correction (S50) de la pression artérielle mesurée de l'utilisateur cible sur la base de la pression mesurée par le capteur de pression et de la taille de poignet de l'utilisateur cible, afin d'obtenir une seconde pression artérielle corrigée de l'utilisateur cible.

8. Procédé selon la revendication 7, dans lequel la correction (S40) de la pression artérielle mesurée de l'utilisateur cible sur la base de la taille de poignet de l'utilisateur cible, afin d'obtenir une première pression artérielle corrigée de l'utilisateur cible comprend :
lorsque la taille de poignet de l'utilisateur cible est supérieure à un premier seuil, la soustraction d'une première valeur spécifiée de la pression artérielle mesurée pour obtenir la première pression artérielle corrigée ; et
lorsque la taille de poignet de l'utilisateur cible est inférieure à un second seuil, l'ajout d'une seconde valeur spécifiée à la pression artérielle mesurée pour obtenir la première pression artérielle corrigée.

9. Procédé selon la revendication 7, dans lequel le bracelet comprend un premier bracelet et un second bracelet qui sont reliés à des extrémités opposées du corps principal, une première partie de boucle est disposée sur le premier bracelet, une pluralité de secondes parties de boucle qui peuvent être fixées à la première partie de boucle sont disposées sur le second bracelet, et la circonférence d'utilisation du dispositif électronique porté au poignet peut être ajustée en fixant différentes secondes parties de boucle dans la pluralité de secondes parties de boucle à la première partie de boucle ; et la mesure (S20) d'une circonférence d'utilisation du dispositif électronique porté au poignet qui s'adapte à une taille de poignet de l'utilisateur cible, et la détermination de la taille de poignet de l'utilisateur cible sur la base de la circonférence d'utilisation du dispositif électronique porté au poignet comprend :
la détection d'une seconde partie de boucle fixée à la première partie de boucle dans la pluralité de secondes parties de boucle, afin de déterminer la circonférence d'utilisation du dispositif électronique porté au poignet.

10. Procédé selon la revendication 9, dans lequel le dispositif électronique porté au poignet comprend une alimentation électrique et une pluralité de résistances de détection disposées sur le second bracelet, au moins une résistance de détection est disposée entre des secondes parties de boucle adjacentes, et lorsque l'une parmi la pluralité de secondes parties de boucle est fixée à la première partie de boucle, une résistance de détection entre la seconde partie de boucle et le corps principal forme une boucle fermée avec l'alimentation électrique ; et
la détection d'une seconde partie de boucle fixée à la première partie de boucle dans la pluralité de secondes parties de boucle, afin de déterminer la circonférence d'utilisation du dispositif électronique porté au poignet comprend :
la détermination, sur la base d'un paramètre physique correspondant à une résistance de la résistance de détection dans la boucle fermée, de la seconde partie de boucle fixée à la première partie de boucle dans la pluralité de secondes parties de boucle.

11. Procédé selon la revendication 7, dans lequel le dispositif électronique comprend une puce de détection disposée dans le corps principal ;
le premier composant de boucle est relié à une broche d'alimentation électrique de la puce de détection ;
chaque second composant de boucle est relié à une broche de détection différente de la puce de détection ;
le procédé comprend en outre :
lorsque le premier composant de boucle est relié à un second composant de boucle cible, la détermination de l'état de niveau de chaque broche de détection de la puce de détection, dans lequel un état de niveau de la broche de détection correspondante qui est reliée au second composant de boucle cible présente un niveau élevé et un état de niveau d'une broche de détection qui n'est pas reliée au second composant de boucle cible présente un niveau bas.
